# EUROPÄISCHE PATENTSCHRIFT

(11) **EP 2 748 591 B1**
(45) Veröffentlichungstag und Bekanntmachung des Hinweises auf die Patenterteilung: **18.11.2020**
(21) Anmeldenummer: 12766904.2
(22) Anmeldetag: 15.08.2012
(51) Int. Cl.: G01N 23/00, G01V 5/00, G01N 33/22

(54) **METHODE ZUM TESTEN UND VALIDIEREN VON PRÜFGERÄTEN**
METHOD OF TESTING AND VALIDATING TEST DEVICES
MÊTHODE POUR TESTER ET VALIDER DES APPAREILS DE CONTRÔLE

(30) Priorität: 22.08.2011 DE 102011081328; 10.11.2011 DE 102011118095
(43) Veröffentlichungstag der Anmeldung: 02.07.2014
(73) Patentinhaber: Smiths Heimann GmbH, 65205 Wiesbaden (DE)
(72) Erfinder: SIEDENBURG, Uwe, 55270 Essenheim (DE)
(74) Vertreter: Greif, Thomas
(86) Internationale Anmeldenummer: PCT/EP2012/065949
(87) Internationale Veröffentlichungsnummer: WO 2013/026752

(56) Entgegenhaltungen:
- D. L. SORBY ET AL: "Dielectric constants of complex pharmaceutical solvent systems. I. Water-ethanol-glycerin and water-ethanol-propylene glycol", JOURNAL OF PHARMACEUTICAL SCIENCES, Bd. 52, Nr. 12, 1. Dezember 1963 (1963-12-01), Seiten 1149-1153, XP55040095, ISSN: 0022-3549, DOI: 10.1002/jps.2600521211
- HERZEN J ET AL: "Quantitative phase-contrast tomography of a liquid phantom using a conventional x-ray tube source", OPTICS EXPRESS OPTICAL SOCIETY OF AMERICA USA, Bd. 17, Nr. 12, 2009, Seiten 10010-10018, XP2686810, ISSN: 1094-4087

## Beschreibung

In der deutschen Patentanmeldung DE 10 2011 081 328.4 ist beschrieben, zur Überprüfung von Personen und Objekten, wie Gepäckstücken, auf gefährliche Stoffe, wie Spreng- oder Explosivstoffe, Prüfanlagen zu verwenden, in denen die zu prüfenden Personen oder Objekte in Prüfgeräte von elektromagnetischen Strahlen durchleuchtet oder bestrahlt werden. Bekannterweise werden derartige Prüfanlagen an Flughäfen zur Überprüfung von Passagieren und Gepäckstücken eingesetzt.

Die Prüfanlagen enthalten nach einer bekannten Ausführungsform Prüfgeräte, bei denen die zu überprüfenden Objekte, beispielsweise die Gepäckstücke, von Röntgenstrahlen durchleuchtet oder bestrahlt werden und die transmittierten oder gestreuten Röntgenstrahlen detektiert und ausgewertet werden (DE 10125531-A, DE 19954662-A).

Zur Überprüfung von Personen sind Prüfgeräte bekannt, bei denen die zu überprüfenden Personen mit elektromagnetischen mm-Wellen bestrahlt und die gestreuten mm-Wellen für eine bildhafte Darstellung ausgewertet werden (DE 102005016106-A).

Vor der Inbetriebnahme müssen die Prüfgeräte getestet und validiert werden. Dies erfolgt üblicherweise mit den realen Gefahrstoffen, also den zu detektierenden Sprengstoffen. Die Benutzung von Sprengstoffen ist gesetzlich geregelt, zudem sind sie schwer handhabbar.

Aus dem US-Patent 5,958,299 ist ein Sprengstoff-Simulationsgemisch bekannt, das nicht explosive Bestandteile enthält, wobei die Komponenten so ausgewählt sind, dass die Mischung eine physikalische Form, Dichte, Röntgentransmission und eine effektive Ordnungszahl aufweist, die einem ausgewählten Sprengstoffgemisch entspricht. Mit dem Simulationsgemisch anstelle eines realen Sprengstoffes lässt sich ein Röntgenprüfgerät auf die Detektion des bestimmten Sprengstoffes testen. Als Simulationsgemische sind feste, plastische und gelförmige Zusammensetzungen beschrieben, die sich aus verschiedenen Komponenten aufbauen.

Die WO 2005/094768 A1 offenbart eine Mischung, die Glycerin und Natriumhydroxid in einem Gewichtsverhältnis von Glycerin zu Natriumhydroxid = 6,25 (20/3,2) enthält.

Die US 2,495,277 A offenbart eine Mischung, die aus 20% Wasser, 15% Natriumhydroxid und 65% Glycerin besteht.

An Prüfanlagen wird zunehmend die Anforderung gestellt, auch Flüssigkeitssprengstoffe und sogenannte "home made explosives" zu detektieren. Der Erfindung liegt daher die Aufgabe zugrunde, eine derartige Spreng- oder Explosivstoffe simulierende Materialmischung bereitzustellen, die nicht explosiv, unkritisch in der Handhabung und preisgünstig in der Herstellung ist, sowie sich in einem Prüfgerät zur Überprüfung von Gegenständen oder Personen verhält wie der reale Gefahrstoff.

In der DE 10 2011 081 328.4 wird als Simulationsgemisch ein Gemisch aus Glycerin, Natriumoxid (NaOH) und Wasser verwendet, wobei Glycerin und Natriumhydroxid in einem Gewichtsverhältnis Glycerin / Natriumhydroxid zwischen 6,5 und 3,8 vorliegen.

Aus der Literatur D. L. SORBY ET AL: "Dielectric constants of complex pharmaceutical solvent systems. I. Water-ethanol-glycerin and water-ethanolpropylene glycol", JOURNAL OF PHARMACEUTICAL SCIENCES, Bd. 52, Nr. 12, 1. Dezember 1963 (1963-12-01), Seiten 1149-1153, XP55040095, ISSN: 0022-3549, DOI: 10.1002/jps.2600521211 oder HERZEN J ET AL: "Quantitative phase-contrast tomography of a liquid phantom using a conventional x-ray tube source", OPTICS EXPRESS OPTICAL SOCIETY OF AMERICA USA, Bd. 17, NR. 12, 2009, Seiten 10010-10018, XP2686810, ISSN: 1094-4087 ist jeweils ein flüssiges Gemisch offenbart, bei dem der Anteil von Glycerin mindestens 90 Gew. % beträgt, und der restliche Anteil Wasser ist.

Nach der Erfindung, die in Anspruch1 definiert ist, wird zur Simulation von explosiven Flüssigkeiten ein flüssiges Gemisch verwendet, bei dem der Anteil von Glycerin mindestens 90 Gew.-%, bevorzugt etwa 99,5 Gew.-% beträgt. Der restliche Anteil ist Wasser.

Mit dieser Mischung werden Prüfgeräte getestet und validiert, die dazu bestimmt sind, flüssige Gefahrstoffe zu detektieren. Bei der Validation wird geprüft, ob die Prüfanlage vorbestimmte Anforderungen erfüllt.

Für den Test und die Validation wird in einem zu überprüfenden Gegenstand oder an einer Person das flüssige Simulationsgemisch nach der Erfindung angeordnet.

In den Prüfgeräten werden die zu überprüfenden Gegenstände, insbesondere Gepäckstücke, mit elektromagnetischen Strahlen durchleuchtet oder bestrahlt. Die transmittierten oder gestreuten Strahlen werden detektiert und ausgewertet.

Eine bevorzugte Verwendung eines Gemischs nach der Erfindung erfolgt zum Testen und Validieren von Röntgenprüfgeräten zur Überprüfung von Personen oder Gegenständen, insbesondere von Gepäckstücken, wie sie in der Beschreibungseinleitung als bekannt beschrieben werden.

Ebenso kann das Simulationsgemisch nach der Erfindung verwendet werden, um Prüfgeräte zu testen und validieren, in denen elektromagnetische mm-Wellen zur Überprüfung von Personen oder Gegenständen verwendet werden.

## Patentansprüche

1. Methode zum Testen und Validieren von Prüfgeräten zur Überprüfung von Gegenständen oder Personen, wobei die Gegenstände oder die Personen mittels elektromagnetischer Strahlung durchleuchtet oder bestrahlt werden, **dadurch gekennzeichnet, dass** ein flüssiges Gemisch, das Glycerin enthält, in einem zu überprüfenden Gegenstand oder an einer Person angeordnet wird, wobei der Anteil von Glycerin mindestens 90 Gew.-%, bevorzugt etwa 99,5 Gew.-%, beträgt, und der restliche Anteil Wasser ist.

2. Methode gemäß Anspruch 1, **dadurch gekennzeichnet, dass** die Prüfgeräte Röntgenprüfgeräte sind.

## Claims

1. Process for testing and validating testing devices for examining objects or persons, wherein the objects or the persons are transilluminated or irradiated by means of electromagnetic radiation, **characterized in that** a liquid mixture containing glycerol is arranged in an object to be examined or on a person, wherein the proportion of glycerol is at least 90 wt%, preferably approximately 99.5 wt%, and the residual proportion is water.

2. Process according to Claim 1, **characterized in that** the testing devices are X-ray testing devices.

## Revendications

1. Méthode pour le test et la validation d'appareils d'essai pour l'inspection d'objets ou de personnes, les objets ou les personnes étant traversé(e)s ou irradié(e)s au moyen d'un rayonnement électromagnétique, **caractérisé en ce qu'**un mélange liquide qui contient de la glycérine est disposé dans un objet devant être inspecté ou est disposé sur une personne, la proportion de glycérine étant d'au moins 90 % poids, préférablement environ 99,5 % en poids, et le reste étant de l'eau.

2. Méthode selon la revendication 1, **caractérisé en ce que** les appareils d'essai sont des appareils d'essai à rayons X.
